# EUROPEAN PATENT SPECIFICATION

(11) **EP 1 292 355 B1**
(45) Date of publication and mention of the grant of the patent: **16.05.2007**
(21) Application number: 01946227.4
(22) Date of filing: 08.06.2001
(51) Int. Cl.: A61M 25/06

(54) **CATHETER AND INTRODUCER NEEDLE ASSEMBLY WITH NEEDLE SHIELD**
KATHETER UND EINFÜHRNADEL-ANORDNUNG MIT NADELSCHUTZ
ENSEMBLE CATHETER ET AIGUILLE D'INTRODUCTION POURVU D'UNE PROTECTION D'AIGUILLE

(30) Priority: 09.06.2000 US 590600; 21.11.2000 US 717148
(43) Date of publication of application: 19.03.2003
(73) Proprietor: Becton Dickinson and Company, Franklin Lakes, New Jersey 07417-1880 (US)
(72) Inventor: HOWELL, Glade, H., Sandy, UT 84094-5470 (US); HARDING, Weston, F., Lehi, UT 84043-3327 (US); CINDRICH, Christopher, N., South Jordan, UT 84095-9601 (US); ERSKINE, Timothy, J., Sandy, UT 84094-7347 (US)
(74) Representative: Ruffles, Graham Keith
(86) International application number: PCT/US2001/018757
(87) International publication number: WO 2001/093940

(56) References cited:
- EP-A- 0 747 083
- EP-A- 0 747 085
- WO-A-98/57689
- WO-A-99/08742
- GB-A- 2 343 118
- US-A- 5 697 907
- US-A- 5 718 688
- US-A- 6 004 294

## Description

### Background of the Invention

The subject invention relates to a catheter and introducer needle assembly that includes a needle shield that will safely shield the sharp distal tip of the introducer needle after the needle has been used to insert the catheter into a patient. In addition, this invention includes a mechanism to connect the needle shield to the catheter until the sharp distal tip of the introducer needle is covered by the needle shield.

Catheters, particularly intravascular (IV) catheters, are used for infusing fluid, such as normal saline solution, various medicaments and total parenteral nutrition, into a patient, withdrawing blood from a patient or monitoring various parameters of the patient's vascular system. Peripheral IV catheters tend to be relatively short, and typically are on the order of about two inches or less in length. The most common type of IV catheter is an over-the-needle peripheral IV catheter. As its name implies, an over-the-needle catheter is mounted over an introducer needle having a sharp distal tip. At least the distal portion of the catheter tightly engages the outer surface of the needle to prevent peelback of the catheter and thus facilitates insertion of the catheter into the blood vessel. The catheter and the introducer needle are assembled so that the distal tip of the introducer needle extends beyond the distal tip of the catheter with the bevel of the needle facing up away from the patient's skin.

The catheter and introducer needle assembly is inserted at a shallow angle through the patient's skin into a blood vessel. There are many techniques for inserting such a catheter and introducer needle assembly into a patient. In one insertion technique, the introducer needle and catheter are inserted completely into the blood vessel together. In another technique, the introducer needle is partially withdrawn into the catheter after the initial insertion into the blood vessel. The catheter is then threaded over the needle and inserted completely into the blood vessel.

In order to verify proper placement of the catheter in the blood vessel, the clinician confirms that there is flashback of blood in a flashback chamber. The flashback chamber is typically formed as part of the needle hub. Once proper placement of the catheter into the blood vessel is confirmed, the clinician applies pressure to the blood vessel by pressing down on the patient's skin over the blood vessel distal of the introducer needle and the catheter. This finger pressure occludes or at least minimizes further blood flow through the introducer needle and the catheter. The clinician then withdraws the introducer needle, leaving the catheter in place, and attaches an appropriate device to the catheter. Such a device can include a fluid delivery device, a PRN, a deadender cap or a blood pressure monitoring probe. Once the introducer needle is withdrawn from the catheter, the introducer needle is a "blood contaminated sharp" and must be properly handled.

In recent years, there has been great concern over the contamination of clinicians with a patient's blood and a recognition that "blood contaminated sharps" must be disposed to avoid an accidental needle stick. This concern has arisen because of the advent of currently incurable and fatal diseases, such as Acquired lmmunosuppressive Deficiency Syndrome ("AIDS"), which can be transmitted by the exchange of body fluids from an infected person to another person. Thus, contact with the body fluid of an AIDS infected person must be avoided. As noted above, if an introducer needle has been used to place a catheter in a blood vessel of an AIDS infected person, the introducer needle, via its sharp distal tip, is a vehicle for the transmission of the disease. Although clinicians are aware of the need to properly handle "blood contaminated sharps", unfortunately in certain medical environments, such as emergency situations or as a result of inattention or neglect, needlesticks with a contaminated introducer needle still occur.

As a result of the problem of accidental needlesticks by "blood contaminated sharps", various needle shields have been developed. Generally, such needle shields work for their intended purpose but could be improved. For example, some needle shields are bulky, difficult to use, require special features or techniques to be operative, or may leave the sharp distal tip exposed after use until the clinician manually activates the needle shielding mechanism.

In addition, some of these needle shields can be easily disconnected from the catheter hub before the needle shield covers the sharp distal tip of the introducer needle. A mechanism to avoid this premature disconnection is a plurality of fingers longitudinally extending from the needle shield with tabs extending radially inwardly from the fingers that engage the flange at the proximal end of the catheter hub. The fingers and tabs hold the needle shield to the catheter. The configuration of the fingers and tabs is designed such that the force needed to overcome the engagement between the fingers and tabs and the catheter hub is greater than the typical force needed to move the introducer needle proximally into the needle shield. However, once the introducer needle has been fully withdrawn into the needle shield, the clinician can exert a greater proximally directed force to remove the needle shield from the catheter hub. Thus the needle shield remains engaged with the catheter until the introducer needle has been completely removed from the catheter and is safely shielded in the needle shield. Unfortunately, this configuration does not consistently ensure that the needle shield remains connected to the catheter hub until the introducer needle is locked in the needle shield. This may be undesirable because the contaminated needle could then be exposed increasing the chances for an accidental needlestick.

GB-A-2343118 (Smiths Industries PLC) describes a needle protector assembly for protecting a needle tip after it has been withdrawn from a catheter. The assembly includes locking arms sprung outwardly away from one another and a trigger lever arranged to engage the needle. When the needle is withdrawn into the housing, the trigger lever disengages the locking arms from one another allowing them to spring outwardly and disengage the catheter arm.

US Patent 5,697,907 (Gaba) describes a safety catheter assembly including a locking arm through which the needle extends, with the locking arm holding the hub of the catheter against the assembly housing. As the needle is withdrawn into the housing, the locking arm is released allowing the hook to separate from the catheter hub.

### Summary of the Invention

It is therefore an object of this invention to provide a needle shield that is compact.

It is another object of this invention to provide a needle shield that is simple and easy to use.

It is still another object of this invention to provide a needle shield that requires no special features or techniques to be operative.

It is yet another object of this invention to provide a needle shield that automatically shields the sharp distal tip of the introducer needle upon withdrawal of the introducer needle from the catheter.

It is a further object of this invention to provide a catheter and introducer needle assembly with a needle shield where the needle shield remains connected to the catheter until the needle shield covers the sharp distal tip of the introducer needle.

According to the present invention, there is provided a catheter and introducer needle assembly as defined in claim 1. Other features of the invention are set forth in the dependent claims.

The catheter and introducer needle assembly with needle shield of this invention includes a catheter, an introducer needle and a needle shield.

The catheter has a distal end and proximal end connected to the distal end of a catheter hub. The catheter is coaxially disposed over the introducer needle and the distal portion of the catheter tightly engages the outer surface of the introducer needle to prevent peelback of the catheter and thus facilitate insertion of the catheter into the patient's blood vessel. Prior to use, the catheter is located about the introducer needle so that the sharp distal tip of the introducer needle is distal of the distal end of the catheter.

The introducer needle has a sharp distal tip and a proximal end connected to the distal end of a needle hub. A flashback chamber may be defined in the needle hub. Typically a vented plug is located in the open proximal end of the flashback chamber to allow air to escape from the flashback chamber when blood enters the flashback chamber from the introducer needle. The introducer needle may also define, along a distal portion thereof, a discontinuous portion, which can take a number of different forms. For example, a slot, or an enlarged diameter portion formed on the introducer needle may be used. If an enlarged diameter portion is used it may have a tapered proximal portion or a tapered distal portion or both. The taper can be oriented at almost any angle to the longitudinal axis of the introducer needle. In certain embodiments, the distal portion may be oriented generally perpendicular to the longitudinal axis of the introducer needle. However, the main requirement is that the discontinuous portion has any configuration that cooperates with a lock associated with the needle shield to prevent unwanted distal movement of the introducer needle with respect to the needle shield. In other words, the sharp distal tip will not be re-exposed from the distal end of the needle shield once the sharp distal tip has been withdrawn into the needle shield after use. The discontinuous portion may also cooperate with a means for preventing unwanted proximal movement of the introducer needle with respect to the needle shield once the sharp distal tip of the introducer needle has been proximally withdrawn into the needle shield. This prevents re-exposure of the sharp distal tip of the introducer needle from the proximal end of the needle shield.

The needle shield is defined by a housing having an internal cavity through which the introducer needle extends. A lock associated with the needle shield prevents unwanted distal movement of the introducer needle once the introducer needle has been proximally withdrawn into the needle shield. Also associated with the needle shield is a means for preventing unwanted proximal movement of the introducer needle once the sharp distal tip of the introducer needle has been proximally withdrawn into the needle shield.

A lock that may be used to prevent unwanted distal movement of the introducer needle once the sharp distal tip of the introducer needle has been proximally withdrawn into the introducer needle shield is a leaf spring. With the leaf spring, any configuration for the discontinuous portion can be used. However, preferably the discontinuous portion is an enlarged diameter portion on the introducer needle with a distally facing shoulder when the leaf spring is used.

The leaf spring has a proximal wall, a support leg and at least one locking leg. Preferably, the support leg and the locking leg are configured such that the locking leg extends generally back toward the proximal end of the support leg. In this configuration the support leg and the locking leg have a generally V-shape, with the apex of the V facing distally. Although this configuration is preferred, any other configuration that biases the locking leg toward the introducer needle could be used. For example, the locking leg could be generally perpendicular to the support leg or oriented at some other angle less than 90 degrees. Alternatively, the locking leg could be formed so it has a generally U-shaped or V-shaped configuration with a pair of spaced apart tines. With such a configuration the locking legs prevent unwanted distal movement of the introducer needle in a manner analogous to the spring gate discussed above. In addition, the portion of the locking leg that rides along the introducer needle shaft could be contoured to approximate a portion of the circumference of the introducer needle to minimize drag as the introducer needle rides past the locking leg.

The configuration of the leaf spring ensures that the locking leg is biased toward and abuts the main portion of the introducer needle. However this bias still allows the locking leg to ride over the enlarged diameter portion as the introducer needle is moved proximally into the needle shield. Once the distally facing shoulder of the enlarged diameter portion is moved proximally of the locking leg, the locking leg moves back into contact with the main portion of the introducer needle. Thereafter, if the introducer needle is moved distally, the locking leg will engage the distally facing shoulder and prevent further distal movement of the introducer needle.

The leaf spring and housing could be configured to maximize the mechanical engagement force between the locking leg and the introducer needle. For example, the cavity where the lock is located could have a tapered cross-section and the leaf spring could be disposed therein so it could move distally into the tapered cross-section.

In this embodiment of the leaf spring, the introducer needle would be proximally withdrawn into the needle shield just like in the previous embodiments until the discontinuous portion is proximal of the locking leg. Thereafter, if the introducer needle is moved distally, the locking leg engages the discontinuous portion and causes the lock to move distally into the tapered cross-section of the cavity. This causes the leaf spring to engage the introducer needle with increasing force until the tapered cross-section prevents further distal movement of the leaf spring. At this point, the locking leg forcefully engages the discontinuous portion preventing distal movement of the introducer needle.

In order to minimize drag on the introducer needle, the locking leg could be initially spaced apart from the introducer needle. In an alternate embodiment, the locking leg could be held out of engagement with the introducer needle by a finger and tab arrangement on the leaf spring and the housing. The leaf spring is movable proximally with respect to the housing by the engagement of the discontinuous portion of the introducer needle with the proximal wall of the leaf spring so the finger and tab can be moved out of engagement with one another. This allows the locking leg to move into engagement with the introducer needle by the inward bias of the locking leg. Thereafter, unwanted distal movement is prevented as discussed above.

In another embodiment of the leaf spring, the cavity has a distal portion and a proximal portion where the diameter of the distal portion is larger than the diameter of the proximal portion. The leaf spring is disposed in the cavity for proximal movement from the distal portion to the proximal portion. When the leaf spring is disposed in the distal portion, the locking leg does not engage the introducer needle. As the introducer needle is withdrawn proximally into the needle shield, the discontinuous portion of the introducer needle engages the proximal wall of the leaf spring. This engagement causes the leaf spring to move proximally with the introducer needle into the proximal portion of the cavity until the proximal wall of the leaf spring abuts the proximal wall of the housing. At this point, any further proximal movement of the leaf spring and the introducer needle is prevented.

When the leaf spring is in the proximal portion, the walls defining the cavity force the locking legs inwardly into engagement with the introducer needle. The leaf spring includes flexible, outwardly extending fingers. The inner walls of the proximal portion in the cavity define slots having a proximally facing wall for receiving the fingers. When the leaf spring is in the proximal portion so the proximal wall of the leaf spring abuts the proximal wall of the housing, the fingers are located in the slots so they are proximal of the proximally facing wall. Thus distal movement of the leaf spring is prevented by the engagement of the fingers and the proximally facing wall. As a result when the discontinuous portion of the introducer needle engages the locking leg, distal movement of the introducer needle is prevented.

Alternatively, the locking leg can be oriented so it is generally perpendicular to the support leg and can be formed with a small diameter opening formed therein. This opening is too small to allow the enlarged diameter portion on the introducer needle to pass therethrough but is large enough to allow the main portion of the introducer needle to pass through.

The leaf spring is held in a biased position by the shaft of the introducer needle. Thus, the locking leg rides along the introducer needle shaft as the introducer needle is retracted into the needle shield. Once the distal end of the introducer needle has been retracted into the needle shield and is proximal of the locking leg, the leaf spring returns to its unbiased position. As such, the locking leg can move so the small diameter opening in the locking leg will be aligned with the sharp distal tip of the introducer needle. Thus, If the introducer needle is moved distally with respect to the leaf spring, the distal end of the introducer needle travels through and past the small diameter opening formed in the locking leg. However, the introducer needle is prevented from being moved distally outside of the needle shield when the enlarged diameter portion on the introducer needle engages the small diameter opening formed in the locking leg.

Preferably, the housing includes at least one additional medial wall adjacent to the locking leg. This medial wall includes an opening therein to allow the main portion of the introducer needle to extend through the medial wall. This provides additional support for the introducer needle and ensures that the introducer needle is aligned with the small diameter opening in the locking leg when the leaf spring returns to its unbiased position.

A variation of the foregoing leaf spring includes an opening in the locking leg with a diameter slightly larger than the diameter of the enlarged diameter portion of the introducer needle. The leaf spring also includes a proximal wall defining an opening therein slightly larger than the diameter of the shaft of the introducer needle but smaller than the diameter of the enlarged diameter portion. The leaf spring is also slidably disposed in the housing of the needle shield but has an end portion of the locking leg that is substantially held in place with respect to the housing. This allows the locking leg to rotate in the housing.

Prior to use when the sharp distal tip of the introducer needle extends beyond the distal end of the needle shield, the locking leg is perpendicular to the longitudinal axis of the introducer needle. In this position, the introducer needle extends through the opening in the proximal wall and the opening in the locking leg. Since the diameter of the opening in the locking leg is slightly larger than the diameter of the enlarged diameter portion, it can be retracted through the opening in the locking leg so the enlarged diameter portion is proximal of the locking leg.

Once the enlarged diameter portion of the introducer needle engages the opening in the proximal wall, the leaf spring moves proximally with the introducer needle until the proximal wall of the leaf spring abuts the proximal wall of the housing. Because one end of the locking leg is substantially held in place with respect to the housing, this proximal movement of the leaf spring causes the proximal leg to rotate clockwise as seen in the FIGS. This changes the orientation of the opening in the locking leg so that a plane defining that opening is no longer perpendicular to the longitudinal axis of the introducer needle. Instead, the opening is angled so that the effective diameter or tne opening, i.e. the perpendicular dimension of the opening, is less than the diameter of the enlarged diameter portion. In addition, in this position, a detent associated with the leaf spring prevents subsequent distal movement of the leaf spring. Thus any subsequent distal movement of the introducer needle is prevented since the enlarged diameter portion can not be moved through the opening in the locking leg.

The means for preventing further proximal movement of the introducer needle once the sharp distal tip of the introducer needle has been proximally withdrawn into the needle shield may take a number of forms. For example, where the discontinuous portion is an enlarged diameter portion, the housing may Include a proximal opening that has a diameter sufficient to allow the main portion of the introducer needle to extend therethrough but that is too small to allow the enlarged diameter portion of the introducer needle from passing therethrough. Alternatively, regardless of the configuration of the discontinuous portion of the introducer needle or where no discontinuous portion is used on the introducer needle, a tether connected to the needle hub and the needle shield could be used.

The needle shield also preferably includes a spring clip that connects the needle shield to the catheter hub and that maintains this connection until the sharp distal tip of the introducer needle has been withdrawn into tne needle shield and locked in place. This spring clip can take many forms.

In one embodiment, the needle shield includes a spring clip that has a clip arm that engages the catheter hub. The spring clip is biased to a position where the clip arm does not engage the catheter hub. The spring clip is held in a biased position where the clip arm engages the catheter hub by the introducer needle shaft. Thus, as long as the introducer needle extends distally past the spring clip, the clip arm remains engaged with the catheter hub and the catheter hub stays connected with the needle shield. Once the sharp distal tip of the introducer needle is withdrawn proximally past the spring clip, the clip arm moves out of engagement with the catheter hub. This allows the catheter hub to be separated from the needle shield. This configuration ensures that the needle shield remains engaged with the catheter until the introducer needle has been completely removed from the catheter and is safely shielded in the needle shield.

The spring clip can be used in combination with any of the looks discussed above for preventing unwanted distal movement of the introducer needle with respect to the needle shield. In addition, the spring clip can be formed integrally with or separately from any of those lacks.

The specific configuration of the spring clip can take many different forms and can be oriented in the housing in many different ways. For example, the spring clip can have a generally V shaped configuration, in this embodiment, the spring clip is located in the housing so it is perpendicular to the longitudinal axis of the introducer needle such that the V configuration is readily apparent from an end cross-sectional view of the needle shield. Thus one leg of the V is held in its biased position, adjacent to the other leg, by direct contact with the shaft of the Introducer needle. In this arrangement, the spring clip includes a clip arm that extends generally parallel to the longitudinal axis of the introducer needle and engages a detent on the catheter hub that is oriented generally perpendicular to the longitudinal axis of the clip arm. When the introducer needle is withdrawn proximal of the spring clip, it returns to its unbiased position such that the clip arm moves transversely, i.e, generally perpendicularly to the longitudinal axis of the introducer needle, out of engagement with the catheter hub.

In an alternate embodiment, the spring clip is generally straight and is flexed into its biased position by the shaft of the introducer needle. In this embodiment, the clip arm has a hook configuration with one leg of the hook extending perpendicular to the longitudinal axis of the introducer needle with the hook facing distally. When the introducer needle is moved proximal of the spring clip, it returns to its unbiased position such that the clip arm moves transversely out of engagement with the catheter hub.

The spring clip can also be generally V shaped but oriented in the housing so it is aligned with the longitudinal axis of the introducer needle such that in a top cross-sectional view of the needle shield, the V configuration is apparent. In this configuration, the clip arm extends generally parallel to the longitudinal axis of the introducer needle and directly engages the catheter hub. However, a separate biasing arm extending generally perpendicular to the longitudinal axis of the introducer needle and connected to each of the legs of the V is required so the introducer needle can bias the spring clip into engagement with the catheter hub. Each biasing arm defines an opening therein and through which the introducer needle extends to achieve this biasing requirement. Once the introducer needle is moved proximal of the biasing arm, the legs of the spring clip can move to their unbiased position out of engagement with the catheter hub.

Where the leaf spring uses a locking leg that is generally perpendicular to the introducer needle, the spring clip can be associated with the locking leg. The spring clip can be pivotally connected to the needle shield housing such that it is caused to pivoted between a clipped position and an undipped position by the movement of the looking leg, Alternatively, the spring clip can be formed as a hook extending distally from the locking leg such that movement of the locking leg from the unshielded to the shielded position causes the hook to move from a clipped position to an undipped position.

With the foregoing embodiments using a biasing arm, a means for minimizing drag on the introducer needle may be used. Such a means is a flap or finger extending from the opening in the biasing arm generally parallel to the longitudinal axis of the introducer needle. Alternatively, where two biasing arms are used, a pin and tether combination can be used. The pin extending through the biasing arms holds the openings in proper alignment so the introducer needle does not catch on the sides of the openings. A tether connected to the pin and the needle hub pulls the pin out of the biasing arms and allows the spring clip to return to its unbiased position.

Where two biasing arms are used, interlocking fingers may be located on the ends of the biasing arms such that, once the introducer needle is withdrawn proximal of the biasing arms, the interlocking fingers lock together to ensure that a non-defeatable transverse barrier is formed by the biasing arms.

With all of the foregoing embodiments, the clip arm can be configured so it engages either the inside or the outside of the catheter hub. In addition, the clip arm can be configured frictionally or mechanically to engage the catheter hub. If a mechanical engagement is desired, the clip arm can have a detent thereon that engages a complementary detent formed on the catheter hub. Complementary detents can include, for example, a slot and a finger, or a post of any geometric shape. Regardless of the specific configuration used, the main requirement is to have the clip arm engage the catheter hub so it is difficult for the clinician to remove the catheter hub from the needle shield until the sharp distal tip of the introducer needle is shielded in the needle shield.

### Brief Description of the Drawings

The preferred embodiments are illustrated in the drawings in which like reference numerals refer to like elements and in which:
FIG. 1 is a perspective view of a standard catheter and introducer needle assembly with the needle shield of this invention with the catheter in partial cross-section;
FIG. 2 is a perspective view of an integrated catheter and introducer needle assembly with the needle shield of this invention;
FIG. 3A is an enlarged elevation view of the distal portion of the introducer needle with one embodiment of the discontinuous portion thereon used in the catheter and introducer needle assembly with the needle shield of this invention;
FIG. 3B is an enlarged elevation view of the distal portion of the introducer needle with a second embodiment of the discontinuous portion thereon used in the catheter and introducer needle assembly with the needle shield of this invention;
FIG. 3C is an enlarged elevation view of the distal portion of the introducer needle with a third embodiment of the discontinuous portion thereon used in the catheter and introducer needle assembly with the needle shield of this invention;
FIG. 3D is an enlarged elevation view of the distal portion the introducer needle with a fourth embodiment of the discontinuous portion thereon used in the catheter and introducer needle assembly with the needle shield of this invention;
FIG. 3E is an enlarged elevation view of the distal portion of the introducer needle with a fifth embodiment of the discontinuous portion thereon used in the catheter and introducer needle assembly with the needle shield of this invention;
FIG. 3F is an enlarged elevation view of the distal portion of the introducer needle with a sixth embodiment of the discontinuous portion thereon used in the catheter and introducer needle assembly with the needle shield of this invention;
FIG. 3G is an enlarged elevation view of the distal portion of the introducer needle with a seventh embodiment of the discontinuous portion thereon used in the catheter and introducer needle assembly with the needle shield of this invention;
FIG. 4 is an exploded perspective view of a catheter, introducer needle and the needle shield with a lock that prevents unwanted distal movement of the introducer needle and a first embodiment of the spring clip that connects the needle shield to the catheter hub until the sharp distal tip of the introducer needle is locked in the needle shield;
FIG. 5 is a partial cross-sectional view of the needle shield with the lock that prevents unwanted distal movement of the introducer needle, the first embodiment of the spring clip that connects the needle shield to the catheter hub until the sharp distal tip of the introducer needle is locked in the needle shield, a portion of the introducer needle and the proximal portion of the catheter hub with the sharp distal tip of the introducer needle extending from the distal end of in the needle shield and the needle shield connected to the catheter hub;
FIG. 6 is a partial cross-sectional view of the needle shield with the lock that prevents unwanted distal movement of the introducer needle, the first embodiment of the spring clip that connects the needle shield to the catheter hub until the sharp distal tip of the introducer needle is locked in the needle shield, the distal portion of the introducer needle and the proximal portion of the catheter hub with the sharp distal tip of the introducer needle locked in the needle shield and the needle shield disconnected from the catheter hub;
FIG. 7A is a perspective view of the first embodiment of the spring clip shown in FIGS. 4 through 6 that connects the needle shield to the catheter hub until the sharp distal tip of the introducer needle is locked in the needle shield;
FIG. 7B is a perspective view of a variation of the first embodiment of the spring clip shown in FIG. 7A that connects the needle shield to the catheter hub until the sharp distal tip of the introducer needle is locked in the needle shield;
FIG. 7C is a perspective view of still another variation of the first embodiment of the spring clip shown in FIG. 7 that connects the needle shield to the catheter hub until the sharp distal tip of the introducer needle is looked in the needle shield;
FIG. 8A is a perspective view of the retention plate that is the lock shown in FIGS. 4 through 6 that prevents unwanted distal movement of the introducer needle;
FIG. 8B is a schematic view, in partial cross-section, of a variation of the retention plate shown in FIG. 8A abutting the proximal face of the medial wall of the housing for the needle shield;
FIG. 8C is a schematic view, in partial cross-section, of still another variation of the retention plate shown in FIG. 8A abutting the proximal face of the medial wall of the housing for the needle shield;
FIG. 9 is a cross-sectional view of the needle shield shown in FIG. 5 taken along line 38-38 showing how the clip arm of the spring clip would move along a ramp formed in the housing;
FIG. 10 is a crose-sectional view of the needle shield shown in FIG. 6 taken along line 39-39 showing how the clip arm of the spring clip would move along a ramp formed in the housing;
FIG. 11 is an exploded perspective view of a catheter, introducer needle, tether and the needle shield with a second embodiment of a spring clip with a transverse barrier that connects the needle shield to the catheter hub until the sharp distal tip of the introducer needle is locked in the needle shield;
FIG. 12 is a cross-sectional view of the needle shield with the second embodiment of the spring clip with a transverse barrier that connects the needle shield to the catheter hub until the sharp distal tip of the introducer needle is locked in the needle shield, a portion of the introducer needle, the tether, the needle shield, and the proximal portion of the catheter hub with the sharp distal tip of the introducer needle extending from the distal end of the needle shield and the needle shield connected to the catheter hub;
FIG. 13 is a cross-sectianal view of the needle shield with the second embodiment of the spring clip with a transverse barrier that connects the needle shield to the catheter hub until the sharp distal tip of the introducer needle is locked in the needle shield, the distal portion of the needle hub, the distal portion of the introducer needle, the tether, and the proximal portion of the catheter hub with the sharp distal tip of the introducer needle locked in the needle shield and the needle shield disconnected from the catheter hub;
FIG. 14 is a perspective view of the second embodiment of the spring clip with a transverse barrier shown in FIGS. 11 through 13;

### Detailed Description of the Invention

As used herein, the term "proximal" refers to a location on the catheter and introducer needle assembly with the needle shield of this invention that, during normal use, is closest to the clinician using the device and farthest from the patient in connection with whom the device is used. Conversely, the term "distal" refers to a location on the catheter and introducer needle assembly of this invention that, during normal use, is farthest from the clinician using the device and closest to the patient in connection with whom the device is used.

As used herein, the term "top", "up" or "upwardly" refers to a location on the catheter and introducer needle assembly with the needle shield of this invention that, during normal use, is radially away from the longitudinal axis of the device and away from the patient's skin. Conversely, as used herein, the term "bottom", "down" or "downwardly" refers to a location on the catheter and introducer needle assembly with the needle shield of this invention that, during normal use, is radially away from the longitudinal axis of the device and toward the patient's skin.

As used herein, the term "in" or "inwardly" refers to a location with respect to the catheter and introducer needle assembly with the needle shield of this invention that, during normal use, is toward the inside of the device. Conversely, as used herein, the term "out" or "outwardly" refers to a location with respect to the catheter and introducer needle assembly with the needle shield of this invention that, during normal use, is toward the outside of the device.

This invention is described herein using like reference numbers for like elements in the different embodiments. Although this invention is described herein in connection with a typical peripheral IV catheter as well as a peripheral IV catheter with an integrated extension tube (an "integrated catheter"), it is to be understood that this invention is applicable to other catheters. For example, this invention is applicable to extended dwell catheters requiring the needle to be connected to the needle hub by a stylet as well as other medical devices where it is desirable for a needle to be shielded after use. In addition, while this invention is satisfied by embodiments in many different forms, there are shown in the drawings and herein described in detail, preferred embodiments of the invention with the scope of the invention measured by the appended claims.

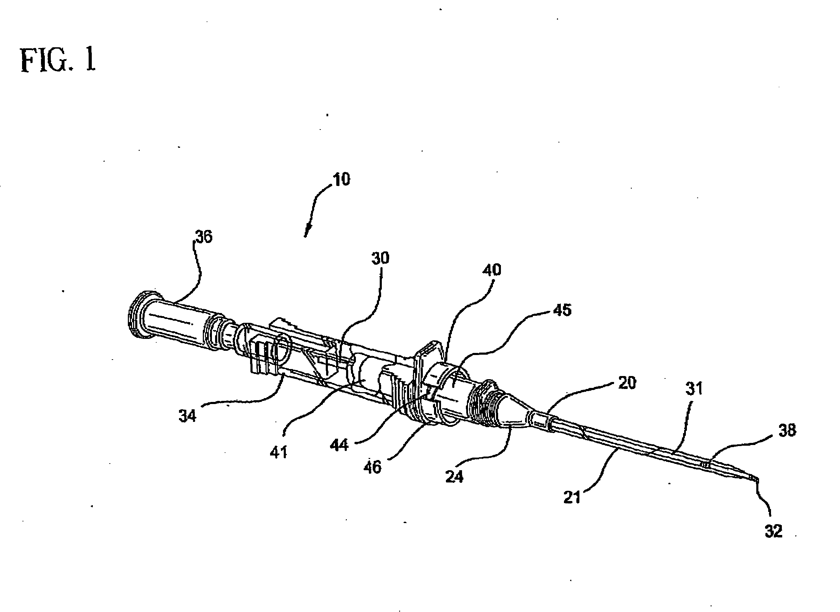

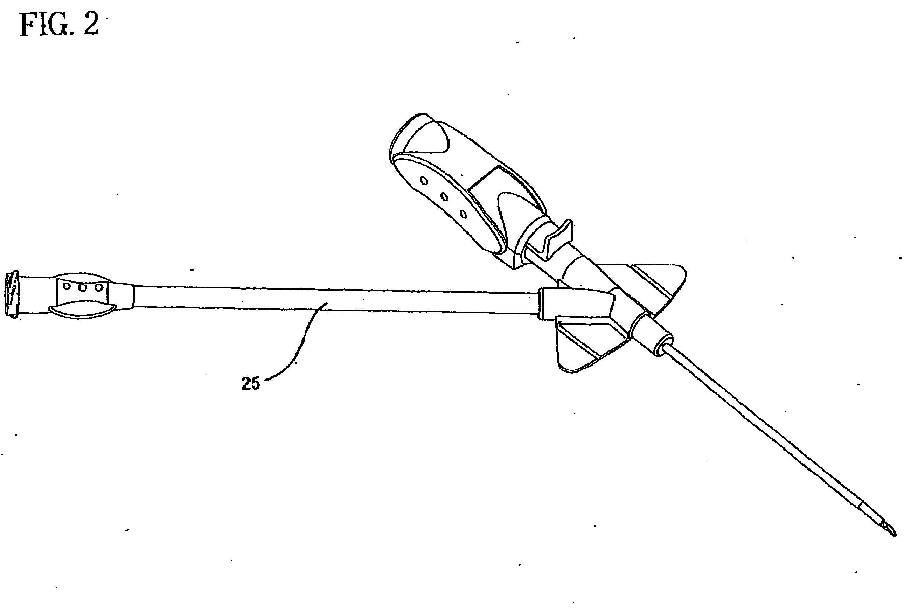

The catheter and introducer needle assembly of this invention is identified generally by the numeral 10 and defines a longitudinal axis extending therethrough. It includes a catheter assembly 20 and an introducer needle assembly 30 that includes a needle shield 40. See FIG. 1. Catheter assembly 20 may include an integrated extension tube 25. See FIG. 2. Such an integrated catheter is described generally in U.S. 5,697,914. As noted above, it is to be understood that the needle shield 40 of this invention can be used in connection with such an integrated catheter.

Catheter assembly 20 includes a catheter 21 that has a proximal end, a distal end and a catheter hub 24 affixed to catheter proximal end. Suitable materials for catheter 21 include, but are not limited to, thermoplastic resins such as fluorinated ethylene propylene (FEP), polytetrafluoroethylene (PTFE), polyurethane and the like. Preferably, catheter 21 is formed from a thermoplastic hydrophilic polyurethane that softens with exposure to physiological conditions present in the patient's body. Suitable materials for catheter hub 24 include, but are not limited to, thermoplastic polymeric resins such as polycarbonate, polystyrene, polypropylene and the like. Catheter hub 24 may include a radially outwardly extending tab, which is useful for advancing catheter 21 into the patient's blood vessel.

Introducer needle assembly 30 includes introducer needle 31 having a sharp distal tip 32 defined by a bevel and a proximal end connected to a needle hub 34. Introducer needle 31 is preferably formed from stainless steel and has a longitudinal axis that is generally parallel to the longitudinal axis of catheter and introducer needle assembly 10. Needle hub 34 can include an integrated flashback chamber having an open proximal end. Needle hub 34 may be formed from the same types of materials that are used to form catheter hub 24. Of course, other materials could be used to form needle hub 34. Preferably, the open proximal end is closed to fluid flow by a vented plug 36 which allows air but not fluid to flow therethrough.

Introducer needle assembly 30 also includes needle shield 40, which includes housing 41 defining an internal cavity 42 therein. Housing 41 also defines a proximal opening 43 and a distal opening 45 in communication with internal cavity 42. This allows introducer needle 31 to extend longitudinally through housing 41. The diameters of cavity 42, proximal opening 43 and distal opening 45 are at least slightly larger than the diameter of the main portion of introducer needle 31. This allows introducer needle 31 easily to pass through needle shield 40. Preferably, cavity 42 and distal opening 45 are at least slightly larger than any discontinuous portion formed on introducer needle 31 as described hereinafter. This allows the distal portion of introducer needle 31 to be withdrawn into housing 41 but prevents introducer needle 31 from being withdrawn proximally from needle shield 40 as described hereinafter.

Where the spring clip, as described hereinafter, of this invention is not used, housing 41 may include a radially extending flange 46 and a plurality of longitudinally extending fingers 47. Fingers 47 may include radially inwardly directed projections 48. Fingers 47 and projections 48 engage catheter hub 24 to hold introducer needle assembly 30 together with catheter assembly 20. Typically, catheter hub 24 includes a radially projecting thread or ear 44 at its proximal end to facilitate the connection of another device thereto. This provides a portion of catheter hub 24 for fingers 47 and projections 48 to engage so that catheter hub 24 and introducer needle assembly 30 remain connected until introducer needle 31 is removed from catheter assembly 20 and is shielded by needle shield 40.

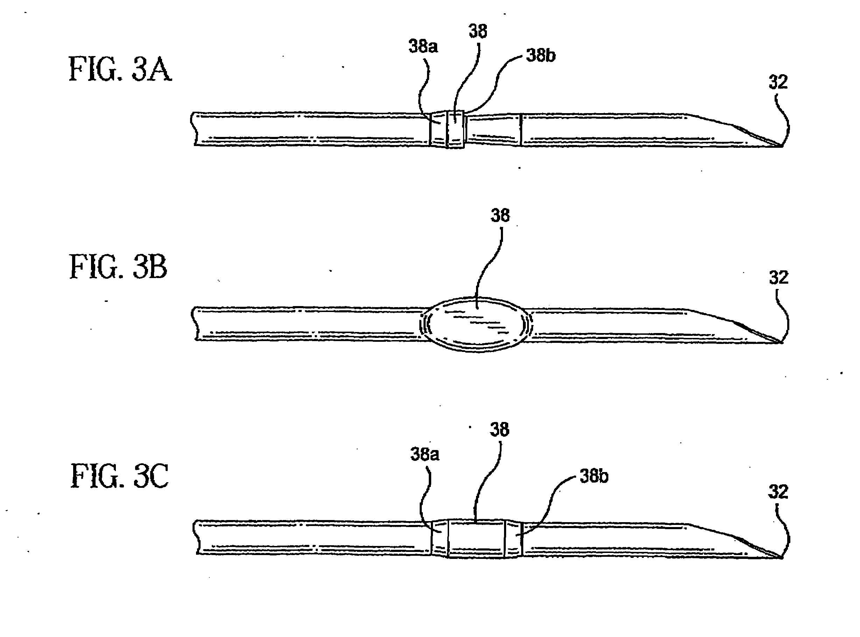

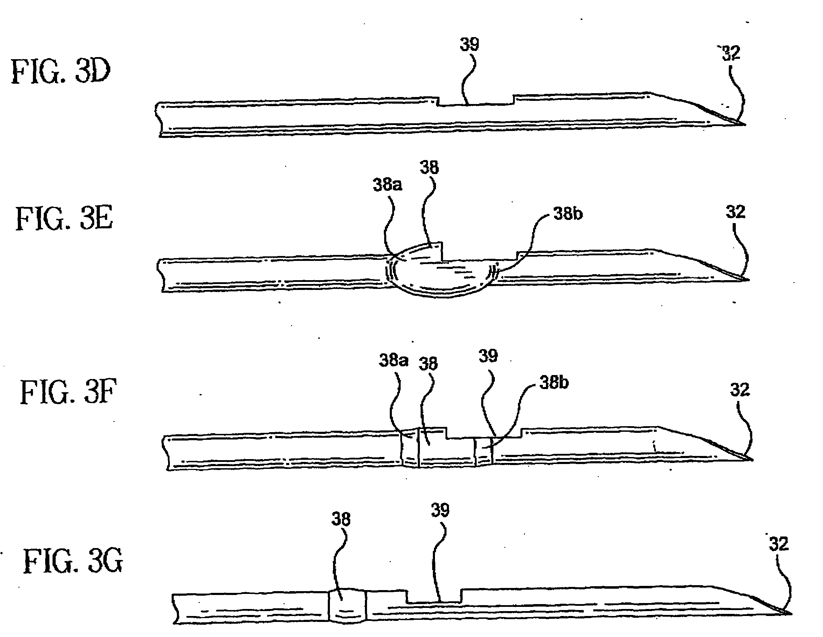

Introducer needle 31 includes a discontinuous portion formed thereon along a distal portion thereof. The discontinuous portion may take many forms. For example, it can be an enlarged diameter portion 38, see FIGS. 3A, 3B, 3C and 3G, or a notch 39, see FIG. 3D and 3G, or a combination of an enlarged diameter portion 38 and a notch 39, see FIGS. 3E, 3F and 3G. Where the discontinuous portion 38 is an enlarged diameter portion, the proximal portion 38a may be tapered and the distal portion 38b may be a distally facing shoulder oriented generally perpendicular to the longitudinal axis of introducer needle 31. See e.g. FIG. 3A. Alternatively, distal portion 38b could be tapered. See e.g. FIG. 3C. This taper can be at just about any angle between almost parallel to and perpendicular to the longitudinal axis of introducer needle. Preferably, enlarged diameter portion 38, such as shown in FIGS. 3A, 3C, 3F and 3G, is formed on introducer needle 31 by centerless grinding a larger diameter introducer needle. Although enlarged diameter portion 38 is shown in the FIGS. as being circumferentially disposed about the shaft of introducer needle 31, it is to be understood, that enlarged diameter portion 38 could be formed asymmetrically about the shaft of introducer needle 31. For example, enlarged diameter portion could be formed as a crimped portion on introducer needle 31. See FIGS. 3B and 3E. For the avoidance of doubt, as used herein the term diameter refers to the length of a straight line passing through the center of an object.

Where the discontinuous portion that is used for introducer needle 31 is enlarged diameter portion 38, it should have a diameter greater than the diameter of proximal opening 43 regardless of the specific configuration used for enlarged diameter portion 38. This provides one mechanism to ensure that introducer needle 31 cannot be pulled in a proximal direction completely out of needle shield 40. This is because enlarged diameter portion 38 blocks further proximal movement of introducer needle 31 through proximal opening 43. Alternatively, a washer 49 having an opening therein with a diameter slightly larger than the diameter of the main portion of introducer needle 31 but smaller than the diameter of enlarged diameter portion 38 can be placed into cavity 42 abutting the proximal wall of cavity 42. In this position, the opening of washer 49 is aligned with proximal opening 43. Thus washer 49 would prevent enlarged diameter portion 38 from passing through proximal opening 43.

Alternatively, if notch 39 were used as the discontinuous portion or if no discontinuous portion were used on introducer needle, a tether 50 could be used to connect needle shield 40 with needle hub 34. See for example FIG. 42. Tether 50 must be configured such that its effective length will not allow sharp distal tip 32 to be pulled proximally completely out of needle shield 40.

The diameter of enlarged diameter portion 38 preferably should be at least about 0.00508cm (0.002 inches) greater than the outside diameter of the main portion of introducer needle 31. It has been surprisingly found that this dimension is sufficient, in the context of this invention, to prevent sharp distal tip 32 of introducer needle 31 from being moved distally out of needle shield 40 after sharp distal tip 32 has been withdrawn proximally into needle shield 40. Where enlarged diameter portion 38 is symmetrically disposed about the shaft of introducer needle 31, preferably the diameter of enlarged diameter portion 38 should be about 0.01016cm (0.004 inches) greater than the diameter of the main portion of introducer needle 31. To ensure this difference in diameter is achieved, that portion of introducer needle 31 immediately distal to distal portion 38b can be formed with a slightly increasing taper from distal portion 38b toward the distal end of introducer needle 31. See FIG. 3A. Grinding that portion of introducer needle 31 can form this taper Immediately distal of distal portion 38b.

A lock that prevents unwanted distal movement of sharp distal tip 32 of introducer needle 31 out of the distal end of needle shield 40 once sharp distal tip 32 has been proximally withdrawn into needle shield 40 can take many forms. As used herein the phrase "unwanted distal movement of introducer needle 31" means distal movement of introducer needle 31, during normal use of catheter and introducer needle assembly 10 and under normal circumstances, such that sharp distal tip 32 is not re-exposed from distal opening 45 of needle shield 40 after the lock engages introducer needle 31.

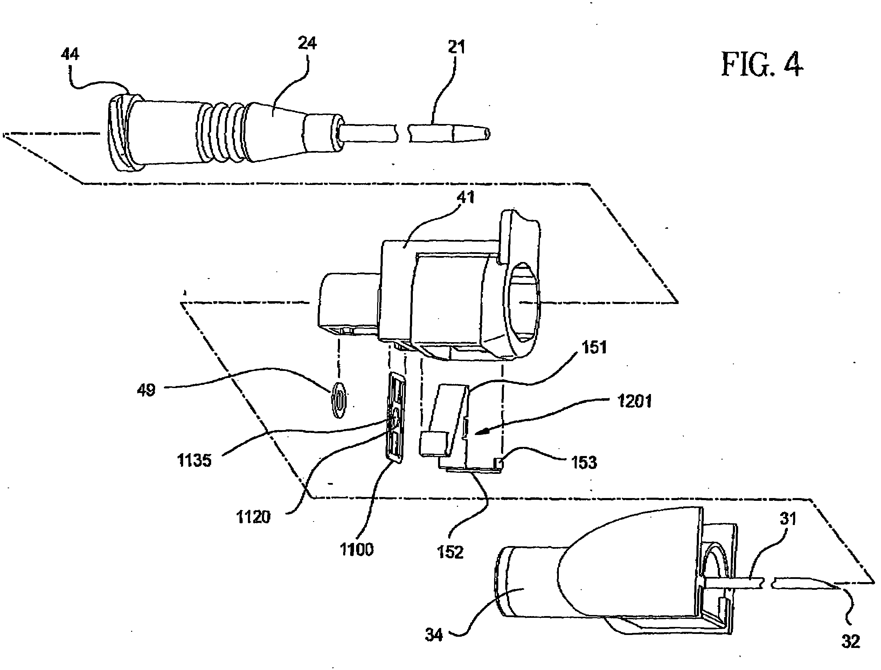

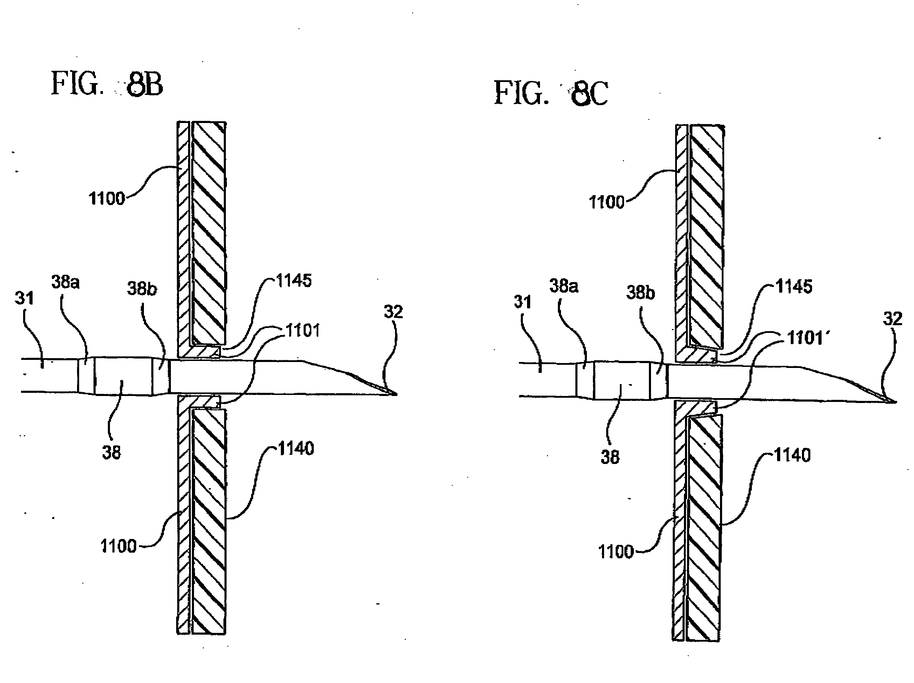

An embodiment of the lock that prevents unwanted distal movement of introducer needle 31 is shown in FIGS. 4 through 6, 8A, 8B and 8C. In this embodiment, the lock is a retention plate 1100, which includes tabs 1120 that do not extend proximally from the main portion of retention plate 1100. See specifically FIG. 8A. However, tabs 1120 are still connected to the main portion of retention plate 1100 via any appropriate mechanism, such as a living hinge, so that tabs 1120 are movable with respect to the main portion of retention plate 1100. Tabs 1120 define a through hole 1130 between the ends of each tab 1120 that has a diameter slightly greater than the diameter of the main portion of introducer needle 31 but is smaller than the diameter of enlarged diameter portion 38. Retention plate 1100 is located adjacent to the proximal face of a medial wall 1140 formed in cavity 42 of housing 41. Medial wall 1140 defines an opening 1145 therethrough that has a diameter greater than the diameter of enlarged diameter portion 38.

As introducer needle 31 Is moved proximally into needle shield 40, enlarged diameter portion 38 can pass through opening 1145. In addition, enlarged diameter portion 38 can move past tabs 1120 since tabs 1120 will move proximally out of the way of enlarged diameter portion 38. However, once enlarged diameter portion 38 is moved proximal of retention plate 1100, any subsequent distal movement of introducer needle 31 with respect to needle shield 40 is prevented. This is because enlarged diameter portion 38 can not extend back through hole 1130 but Instead engages the surface of tabs 1120 adjacent to hole 1130. In addition, tabs 1120 cannot move distally out of the way of enlarged diameter portion 38 because medial wall 1140 prevents any such distal movement of tabs 1120. Any further proximal movement of introducer needle 31 is prevented by proximal opening 43, washer 49 or tether 50.

FIGS. 8B and 8C show variations of the retention plate shown in FIG. 8A. In both of these variations, tabs 1120 include distally extending fingers adjacent to hole 1130 such as to define the diameter of hole 1130. Fingers 1101 are shown in FIG. 8B and fingers 1101' are shown in FIG. 8C. in FIG. 8C, the outer portions of fingers 1101' are tapered so they slope toward introducer needle 31 in the distal direction and the inner walls defining opening 1145 have a complementary slope. Fingers 1101 and 1101' fill any excess space in opening 1145 in medial wall 1140 and ensure that unwanted distal movement of introducer needle 31. In the case of the variation shown in FIG. 8C, fingers 1101' provide a greater holding force to introducer needle 31 to prevent unwanted distal movement of introducer needle 31. This is because as introducer needle 31 is moved distally after enlarged diameter portion 38 has been moved proximal of retention plate 1100, fingers 1101' will wedge Into opening 1145 making it significantly more difficult to move introducer needle 31 distally.

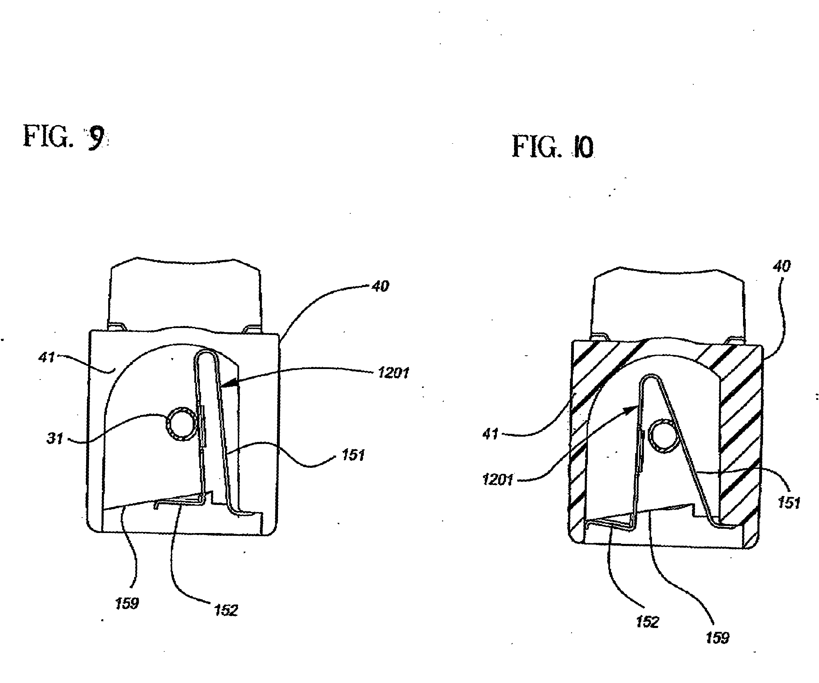

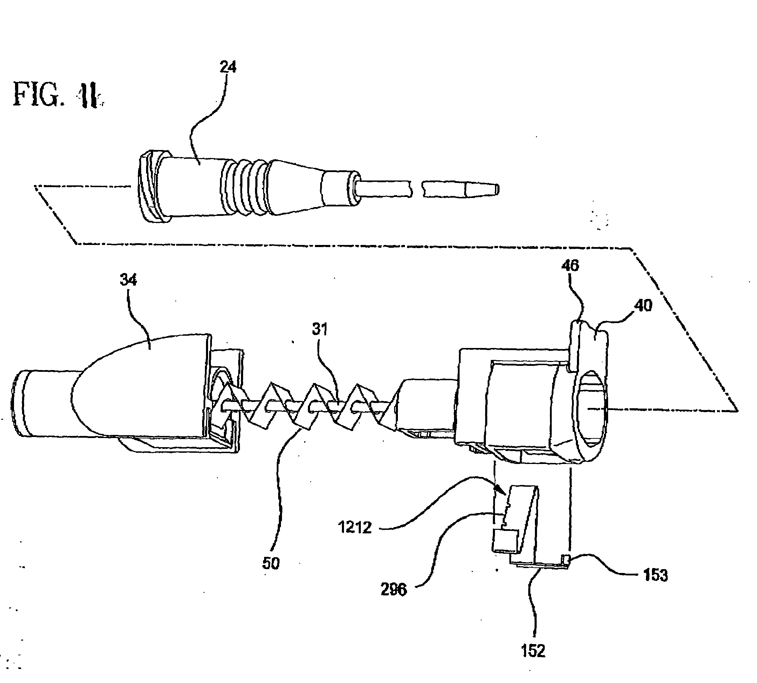

FIGS. 4 through 7C, 9 and 10 show a first embodiment of a resilient spring clip 1201 that is used to connect needle shield 40 to catheter hub 24 until sharp distal tip 32 of introducer needle 31 has been withdrawn into needle shield 40. A spring arm 151 that is formed in a V-shaped configuration defines the spring clip. Spring arm 151 is disposed in housing 41 such that the apex of the V is pointed up toward the top of housing 41 and the legs defining the V straddle the longitudinal axis of Introducer needle 31 when spring arm 151 is in its unbiased position. In this orientation, spring arm 151 is adapted for motion transverse to the longitudinal axis of introducer needle 31. This motion is along a defined path provided by a ramped surface 159 In housing 41. See FIGS. 9 and 10.

Of course spring arm 151 could be disposed in housing 41 such that the apex is oriented in other positions on a circle concentric to the longitudinal axis of introducer needle 31. All that is required is that spring arm 151 be adapted for motion transverse to the longitudinal axis of introducer needle 31. The apex of the V shape facilitates the flexing of spring arm 151 to and from a biased condition. In addition, the apex of the V shape could be in the form of a living hinge.

When sharp distal tip 32 of introducer needle 31 is distal of needle shield 40, introducer needle 31 abuts spring arm 151 so as to hold spring arm 151 in the biased, unactivated, clipped position. Spring arm 151 includes a clip arm 152 which extends generally parallel to the longitudinal axis of introducer needle 31. Clip arm 152 preferably has a finger 153 formed thereon, which is adapted to engage thread 44 or a corresponding detent 26 formed on catheter hub 24 when spring arm 151 is in the clipped position. Detent 26 can be the flange or luer locking ears 44 shown on the proximal end of catheter hub 24. Alternatively, detent 26 can take the form of a notch or slot, or an upstanding peg. Although the use of finger 153 and detent 26 is preferred because it provides positive mechanical engagement therebetween, these elements are not necessary. Without any detents the engagement force between clip arm 152 and catheter hub 24 is limited to frictional force which may be easier to overcome than with mechanical engagement However, in certain situations, this frictional force may be sufficient.

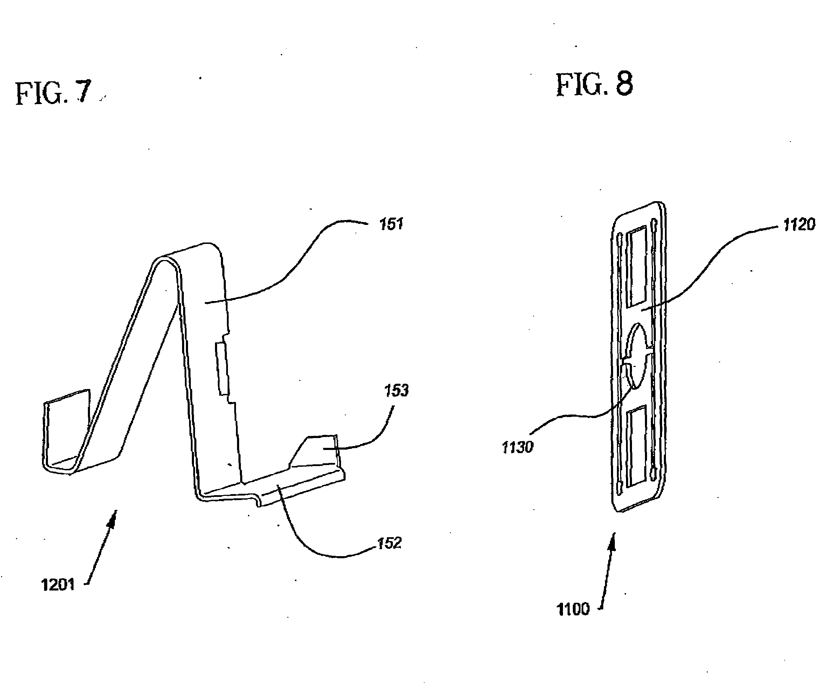

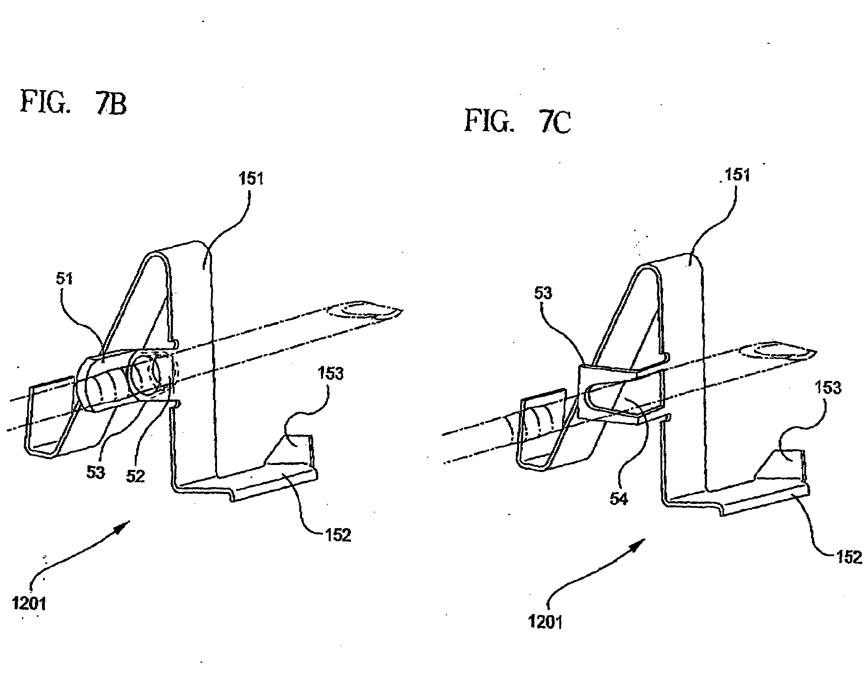

When sharp distal tip 32 of introducer needle 31 is moved proximally into needle shield 40 so that introducer needle 31 no longer abuts spring arm 151, spring arm 151 can flex to its unbiased, activated non-clipped position out of engagement with catheter hub 24. This allows catheter hub 24 to be disconnected from needle shield 40. Spring arm 151 may include a longitudinally extending flog 158 that minimizes drag on introducer needle 31, and enlarged diameter portion 38 if any, as introducer needle 31 is being moved proximally into needle shield 40. In addition, spring arm 151 may include an arm 53 extending therefrom and which defines an opening therein. In one embodiment, arm 53 also includes a longitudinally extending guide rail 51 that guides introducer needle 31 toward opening 52. See FIG. 7B. The diameter of opening 52 is slightly greater than the diameter of the main portion of introducer needle 31 but is smaller than the diameter of enlarged diameter portion 38. Thus spring clip 151 can work to prevent unwanted distal movement of introducer needle 31. In an alternative embodiment a tapered opening 54 can be used instead. See FIG. 7. All that is required is for opening 54 to define a portion that allows the main portion of introducer needle 31 to extend therethrough and to define a portion that does not allow the enlarged diameter portion 38 to extend therethrough.

It is to be understood that the various embodiments of the resilient spring clips discussed hereinafter can be used in conjunction with any of the previous embodiments of the lock that prevents unwanted distal movement of sharp distal tip 32 of introducer needle 31 out of the distal end of needle shield 40 once sharp distal tip 32 has been proximally withdrawn into needle shield 40. Preferably, the resilient spring clips are formed from stainless steel. However, it is to be understood that other flexible, strong materials could also be used to form the resilient spring clips.

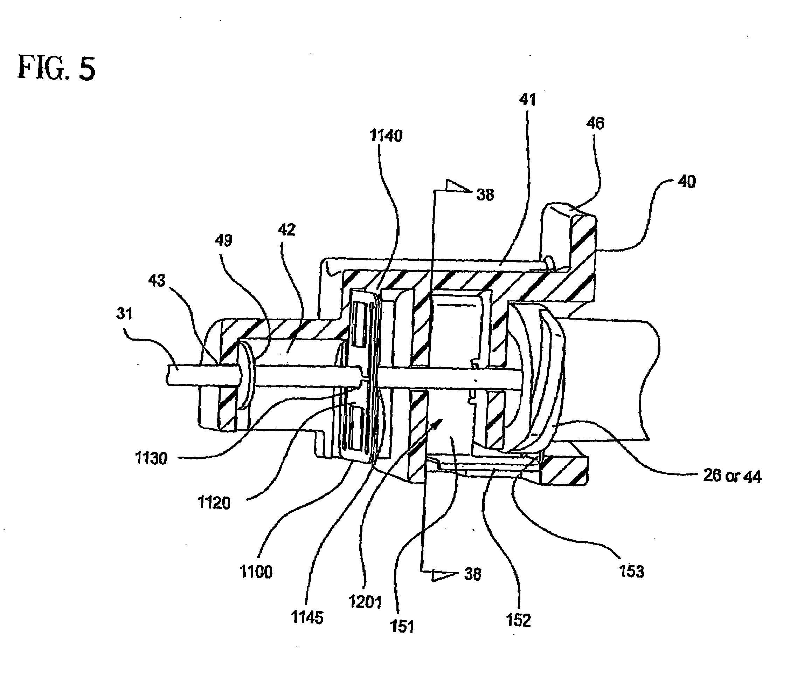

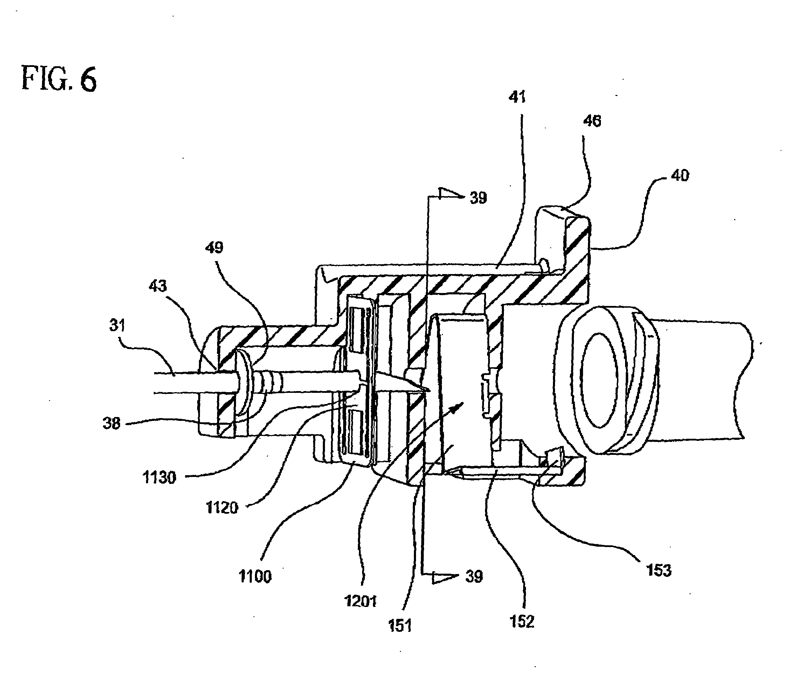

As previously mentioned, the V-shaped configuration of spring arm 151 ensures that it can flex between a clipped, i.e. a biased and an unactivated, position and a non-clipped, i.e. an unbiased and an activated, position. In the unactivated position, finger 153 is positioned into engagement with catheter hub 24. See FIG. 5. In the activated position, finger 153 is not in engagement with catheter hub 24. See FIG. 6. Preferably, housing 41 defines a ramp 159 extending transversely to the longitudinal axis of introducer needle 31. See FIGS. 9 and 10. Ramp 159 is located in housing 41 such that it engages clip arm 152. Ramp 159 acts as a guide for clip arm 152 to ensure clip arm 152 and finger 153 do not rotate around catheter hub 24 but instead move transversely to the longitudinal axis of introducer needle 31 once sharp distal tip 31 is withdrawn proximally of spring arm 151 out of engagement with catheter hub 24.

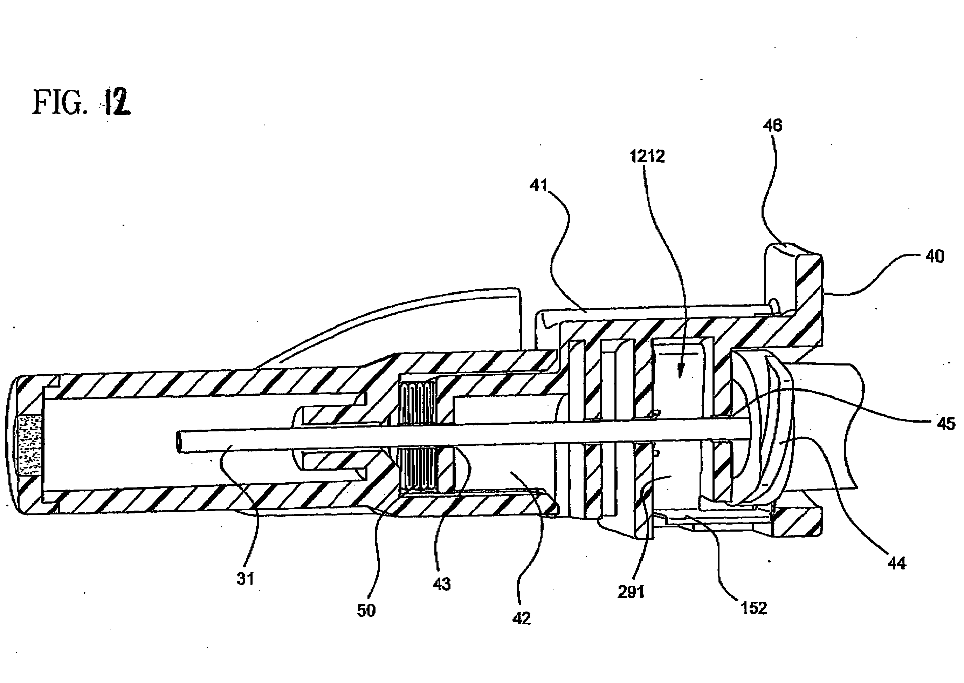

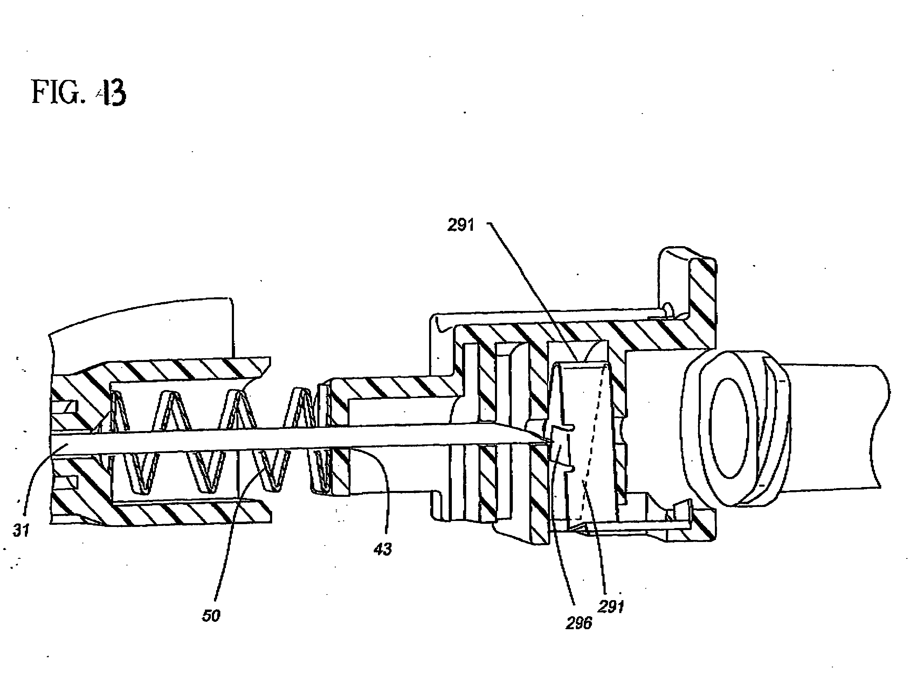

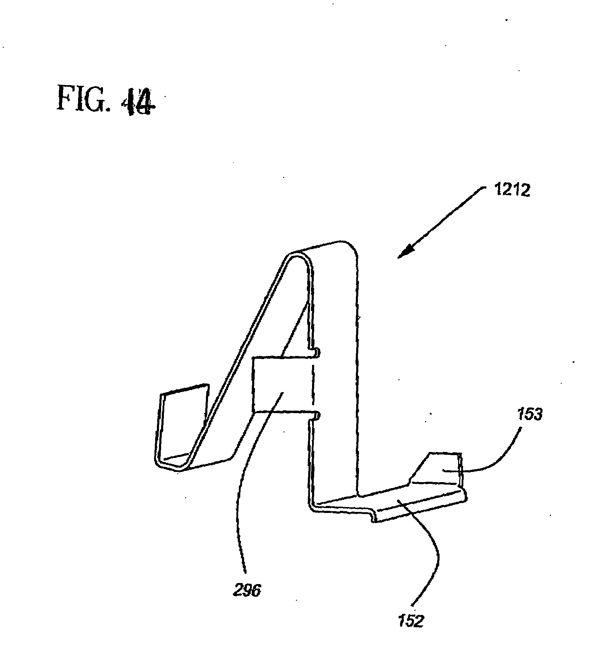

FIGS. 11 through 14 show a second embodiment of a spring clip 1212 with a transverse barrier that connects catheter hub 24 to needle shield 40 until sharp distal tip 32 has been locked in needle shield 40. Spring clip 1212 of this embodiment is substantially the same as the embodiment for the spring clip shown in FIGS. 4 through 7C, 9 and 10 except for the addition of transverse barrier 296. Thus this embodiment of spring clip 1212 functions to connect catheter hub 24 to needle shield 40 until sharp distal tip 32 has been locked in needle shield 40 in substantially the same way as the embodiment shown in FIGS. 4 through 7C, 9 and 10.

When introducer needle 31 extends through needle shield 40 so that sharp distal tip 32 is distal of distal opening 45, spring arm 291 abuts the shaft of introducer needle 31 and is biased to move transverse barrier 296 in front of sharp distal tip 32. Once sharp distal tip 32 is moved proximally past spring arm 291, spring arm 291 moves to Its activated, unbiased, non-clipped position so that transverse barrier 296 is in front of sharp distal tip 32. This prevents any unwanted distal movement of introducer needle 31.

Where transverse barrier 296 is used, preferably a tether 50 is used to connect needle shield 40 to needle hub 34. Tether 50 prevents unwanted proximal movement of introducer needle 31 with respect to needle shield 40. Tether 50 can take many different forms such as a string, a pleated element, a sleeve member surrounding introducer needle 31 or a plurality of telescoping members surrounding introducer needle 31. Alternatively, where transverse barrier 296 is used, unwanted proximal movement of introducer needle 31 can be prevented in the same manner as the embodiments previously discussed. As such, introducer needle 31 can be formed with enlarged diameter portion 38, which would abut proximal opening 43 or washer 49.

Where enlarged diameter portion 38 is used on introducer needle 31, spring clip 1212 could be formed as shown in FIGS. 7B or 7C. As discussed above, in those embodiments, the spring clip is formed with an arm 53 extending therefrom similarly to transverse barrier 296. However, arm 53 defines an opening 52 or 54 therein. Opening 54 is tapered from a larger diameter portion to a smaller diameter portion. In both cases, opening 52 and 54 define a portion that has a diameter that is smaller than the diameter of enlarged diameter portion 38. Thus, after spring clip 1212 moves to the non-clipped position, opening 52 and 54 will be substantially aligned with introducer needle 31. However, unwanted distal movement of introducer needle 31 will be prevented because the smaller diameter portion of opening 52 and 54 will prevent the passage of enlarged diameter portion 38 therethrough. Similarly, opening 199 shown in the embodiment of FIG. 14 can cooperate with enlarged diameter portion 38 to prevent unwanted distal movement of introducer needle 31.

In order to place catheter 21 into a patient's blood vessel, the clinician substantially longitudinally aligns introducer needle 31 and catheter 21 with the target blood vessel. The bevel of sharp distal tip 32 should be facing substantially away from the skin surface during venipuncture. The cliniclan inserts introducer needle 31 and catheter 21 at a shallow angle, preferably less than about 35 degrees, into the skin so that sharp distal tip 32 enters the target blood vessel. The clinician then preferably observes a blood flashback in the flashback chamber of needle hub 34.

After confirming placement of introducer needle 31 and catheter 21 in the target blood vessel, the clinician advances catheter 21 distally axially along introducer needle 31 into position in the blood vessel. In certain techniques, introducer needle 31 may be partially withdrawn into catheter 21 before catheter 21 is completely advanced into position in the blood vessel. After proper placement of catheter 21 is achieved, the clinician places a finger from her other hand on the patient's skin over the blood vessel approximately over the distal end of catheter 21. By placing her finger on the patient's skin and applying sufficient pressure on the skin, the clinican thereby substantially occludes or at least minimizes blood flow through catheter 21. The clinican then withdraws introducer needle 31 completely from catheter 21 by moving needle hub 34 proximally. This movement causes introducer needle 31 to move proximally into needle shield 40.

Where one of the embodiments of the spring clip disclosed herein to connect needle shield 40 to catheter hub 24 until sharp distal tip 32 of introducer needle 31 has been withdrawn into needle shield 40 is used, needle shield 40 remains engaged with catheter hub 24 during this proximal movement of introducer needle 31. Once sharp distal tip 32 of introducer needle 31 has been withdrawn into needle shield 40 so that the lock engages introducer needle 31 to prevent unwanted distal movement of sharp distal tip 32 of introducer needle 31 out of the distal end of needle shield 40, needle shield 40 can be disconnected from catheter hub 24. After introducer needle 31 and needle shield 40 have been removed from catheter hub 24, the clinician may then attach a fluid delivery device, a PRN, a deadender cap or some other blood monitoring device to catheter hub 24 and commence the planned treatment. Introducer needle 31 and needle shield 40 may then be disposed of according to the facility's disposal protocol.

Thus, it is seen that a catheter and introducer needle assembly with needle shield is provided that is compact, simple and easy to use, that requires no special features or technique to be operative, that automatically shields the sharp distal tip of the introducer needle upon withdrawal of the introducer needle from the catheter and where the needle shield remains connected to the catheter until the needle shield covers the sharp distal tip of the introducer needle.

## Claims

1. A catheter and introducer needle assembly, comprising:
a catheter (21) having a proximal end and a distal end;
a catheter hub (24) in fluid communication with the catheter and having a proximal end and a distal end connected to the proximal end of the catheter;
an introducer needle (31) disposed in the catheter and having a proximal end and a distal end and an axis extending from the proximal end to the distal end; and
a needle shield (40) having a proximal portion and a distal portion and including a resilient clip (1201) adapted to flex between a biased position and an unbiased position in a plane substantially perpendicular to the axis of the introducer needle;
wherein, in the biased position, the clip engages the catheter hub and in the unbiased position the clip disengages the catheter hub;
wherein when the distal end of the introducer needle extends from the distal portion of the needle shield, the clip is held to one side of the introducer needle so it is flexed into the biased position and when the sharp distal end of the introducer needle is withdrawn into the needle shield, the clip flexes to the unbiased position out of engagement with the catheter hub;
**characterised in that** the clip has a generally V-shaped configuration, and in the biased position one leg of the V is held in its biased position adjacent to the other leg by direct contact with the shaft of the introducer needle.

2. The catheter and introducer needle assembly of claim 1 wherein the clip (1201) includes a spring arm (151) and at least one clip arm (152) that extends from the spring arm.

3. The catheter and introducer needle assembly of claim 2 wherein the clip arm includes a finger (153) thereon.

4. The catheter and introducer needle assembly of claim 3 wherein the finger (153) mechanically engages the catheter hub (24) to hold the catheter hub to the needle shield (40) when the introducer needle contacts the clip.

5. The catheter and introducer needle assembly of claim 4 wherein the catheter hub (24) includes an engagement member (44) which is mechanically engaged by the finger when the introducer needle contacts the clip so the catheter hub is connected to the needle shield until the distal end of the introducer needle is withdrawn into the needle shield.

6. The catheter and introducer needle assembly of claim 5 wherein the engagement member (44) is a thread or detent.

7. The catheter and introducer needle assembly of claim 1 wherein the clip (1201) includes a transverse barrier (196) extending therefrom that acts as a mechanical barrier to prevent unwanted distal movement of the introducer needle with respect to the needle shield.

## Patentansprüche

1. Katheter und Einführnadelanordnung, umfassend:
Einen Katheter (21) mit einem proximalen Ende und einem distalen Ende;
eine Katheternabe (24) in Flüssigkeitskommunikation mit dem Katheter und ein proximales Ende und ein distales Ende aufweisend, das mit dem proximalen Ende des Katheters verbunden ist;
eine Einführnadel (31), die im Katheter angeordnet ist und ein proximales Ende und ein distales Ende und eine Achse aufweist, die sich vom proximalen Ende zum distalen Ende erstreckt; und
einen Nadelschutz (40) mit einem proximalen Teil und einem distalen Teil und einem Federclip (1201), der angepasst ist, sich flexibel zwischen einer vorgespannten Position und einer nicht vorgespannten Position in einer Ebene zu bewegen, die im Wesentlichen senkrecht zur Achse der Einführnadel ist;
wobei der Clip, in der vorgespannten Position, die Katheternabe in Eingriff bringt und der Clip, in der nicht vorgespannten Position, die Katheternabe außer Eingriff bringt;
wobei, wenn sich das distale Ende der Einführnadel aus dem distalen Teil des Nadelschutzes erstreckt, wird der Clip zur einen Seite der Einführnadel gehalten, damit er in die vorgespannte Position gebogen wird und, wenn das scharfe distale Ende der Einführnadel in den Nadelschutz zurückgezogen wird, biegt sich der Clip in die nicht vorgespannte Position, außer Eingriff mit der Katheternabe;
**dadurch gekennzeichnet, dass** der Clip generell eine V-förmige Konfiguration aufweist und in der vorgespannten Position ein Schenkel des Vs in seiner vorgespannten Position angrenzend an den anderen Schenkel durch direkten Kontakt mit dem Schaft der Einführnadel gehalten wird.

2. Katheter und Einführnadelanordnung nach Anspruch 1, wobei der Clip (1201) einen Federarm (151) und mindestens einen Cliparm (152) umfasst, der sich vom Federarm erstreckt.

3. Katheter und Einführnadelanordnung nach Anspruch 2, wobei der Cliparm einen Finger (153) daran umfasst.

4. Katheter und Einfühmadelanardnung nach Anspruch 3, wobei der Finger (153) mechanisch in die Katheternabe (24) einrastet, um die Katheternabe an den Nadelschutz (40) zu halten, wenn die Einführnadel den Clip kontaktiert.

5. Katheter und Einführnadelanordnung nach Anspruch 4, wobei die Katheternabe (24) ein Einrastelement (44) umfasst, in das der Finger mechanisch einrastet, wenn die Einführnadel den Clip kontaktiert, damit die Katheternabe mit dem Nadelschutz verbunden ist, bis das distale Ende der Einführnadel in den Nadelschutz zurückgezogen ist.

6. Katheter und Einführnadelanordnung nach Anspruch 5, wobei das Einrastelement (44) ein Gewinde oder eine Arretierung ist.

7. Katheter und Einführnadelanordnung nach Anspruch 1, wobei der Clip (1201) einen sich daraus erstreckenden Querriegel (196) umfasst, der als eine mechanische Sperre fungiert, um unerwünschte distale Bewegung der Einführnadel in Bezug auf den Nadelschutz zu verhindern.

## Revendications

1. Assemblage de cathéter et d'aiguille d'introduction, comprenant:
un cathéter (21), comportant une extrémité proximale et une extrémité distale ;
un moyeu de cathéter (24), en communication de fluide avec le cathéter et comportant une extrémité proximale et une extrémité distale connectée à l'extrémité proximale du cathéter ;
une aiguille d'introduction (31) agencée dans le cathéter et comportant une extrémité proximale et une extrémité distale et un axe s'étendant de l'extrémité proximale vers l'extrémité distale ; et
un protecteur d'aiguille (40), comportant une partie proximale et une partie distale et englobant une bride élastique (1201) destinée à être fléchie entre une position sollicitée et une position non sollicitée dans un plan pratiquement perpendiculaire à l'axe de l'aiguille d'introduction;
la bride s'engageant dans la position sollicitée dans le moyeu de cathéter et la bride étant dégagée du moyeu du cathéter dans la position non sollicitée ;
la bride étant retenue vers un côté de l'aiguille d'introduction lorsque l'extrémité distale de l'aiguille d'introduction s'étend à partir de la partie distale du protecteur d'aiguille, de sorte à être fléchie dans la position sollicitée, la bride étant fléchie vers la position non sollicitée et dégagée du moyeu du cathéter lorsque l'extrémité distale tranchante de l'aiguille d'introduction est rétractée dans le protecteur d'aiguille ;
**caractérisé en ce que** la bride a généralement une configuration en forme de V et que dans 1a position sollicitée une jambe du V est retenue dans sa position sollicitée adjacente à l'autre jambe par contact direct avec la tige de l'aiguille d'introduction.

2. Assemblage de cathéter et d'aiguille d'introduction selon la revendication 1, dans lequel la bride (1201) englobe un bras à ressort (151), au moins un bras de la bride (152) s'étendant à partir du bras à ressort.

3. Assemblage de cathéter et d'aiguille d'introduction selon la revendication 2, dans lequel le bras de la bride englobe un doigt (153) qui y est agencé.

4. Assemblage de cathéter et d'aiguille d'introduction selon la revendication 3, dans lequel le doigt (153) s'engage de manière mécanique dans le moyeu de cathéter (24) pour retenir le moyeu de cathéter sur le protecteur d'aiguille (40) lorsque l'aiguille d'introduction contacte la bride.

5. Assemblage de cathéter et d'aiguille d'introduction selon la revendication 4, dans lequel le moyeu du cathéter (24) englobe un élément d'engagement (44), engagé mécaniquement dans le doigt lorsque l'aiguille d'introduction contacte la bride, de sorte que le moyeu du cathéter est connecté au protecteur d'aiguille jusqu'à la rétraction de l'extrémité distale de l'aiguille d'introduction dans le protecteur d'aiguille.

6. Assemblage de cathéter et d'aiguille d'introduction selon la revendication 5, dans lequel l'élément d'engagement (44) est constitué par un filetage ou un cliquet.

7. Assemblage de cathéter et d'aiguille d'introduction selon la revendication 1, dans lequel la bride (1201) englobe une barrière transversale (196) à extension, servant de barrière mécanique pour empêcher un déplacement distal intempestif de l'aiguille d'introduction par rapport au protecteur d'aiguille.
